# EUROPÄISCHE PATENTSCHRIFT

(11) **EP 2 953 674 B1**
(45) Veröffentlichungstag und Bekanntmachung des Hinweises auf die Patenterteilung: **14.06.2017**
(21) Anmeldenummer: 14707091.6
(22) Anmeldetag: 05.02.2014
(51) Int. Cl.: A61M 16/04, A61F 13/02, A61M 16/10, A61M 16/20

(54) **TRACHEOSTOMAPFLASTER**
TRACHEOSTOMA PLASTER
PANSEMENT POUR TRACHÉOTOMIE

(30) Priorität: 05.02.2013 DE 102013001910
(43) Veröffentlichungstag der Anmeldung: 16.12.2015
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2014/000301
(87) Internationale Veröffentlichungsnummer: WO 2014/121921

(56) Entgegenhaltungen:
- EP-A2- 2 497 449
- DE-T2- 60 312 327
- DE-U1-202006 005 101
- DE-U1-202008 017 105
- US-A1- 2003 088 219
- US-A1- 2012 101 458

## Beschreibung

Die vorliegende Erfindung betrifft ein Tracheostomapflaster mit einer proximalen, dem Körper zugewandten, und einer distalen, dem Körper abgewandten, Seite und einem Klebeband.

Tracheostomapflaster der eingangs genannten Art dienen zur Befestigung von Tracheostomahilfsmitteln wie Feuchte-/Wärmetauschern (HME (Humid Moisture Exchanger) genannt) oder Sprechventilen, die zugleich eine HME-Funktion ausüben, am Tracheostoma eines Tracheotomierten. Eine Tracheostoma ist eine künstliche Atemöffnung, die bei operativen Eingriffen am oberen Atemtrakt unter Umständen angelegt werden muss, damit unter Umgehung von Mundraum und Kehlkopf Luft direkt in die Lunge eingeatmet werden kann. Bei Tracheotomierten werden üblicherweise Filtersysteme eingesetzt, welche dazu dienen, die bei Tracheotomierten fehlenden Regulationsmechanismen zur Erwärmung und Befeuchtung der Atemluft nachzubilden und ein Inkontaktbringen der Luftröhre mit trockener, kalter und nicht gefilterter Luft zu vermeiden. Hierdurch werden Reizungen und damit auch eine erhöhte Schleimproduktion als auch die Gefahr einer Verborkung vermieden. Durch solche Filtersysteme, die auch künstliche Nasen genannt werden, wird die eingeatmete Luft angefeuchtet, erwärmt und gleichzeitig gefiltert. Das regelmäßige Tragen der künstlichen Nase hilft insbesondere auch bei starker Sekretabsonderung, da durch das Anfeuchten der Schleimhäute in der Luftröhre die Sekretproduktion vermindert wird.

Die vorstehend genannten Filtersysteme können auch mit einer Sprechfunktion ausgestattet sein, wie diese beispielsweise in der EP 1 747 792 A1 offenbart ist. Der dort offenbarte Feuchte- und Wärmeaustauscher mit Sprechfunktion weist ein Filtergehäuse und einen in diesem zumindest teilweise angeordneten Filterkörper auf, wobei das Filtergehäuse ein auf seiner distalen Seite angeordnetes Sprechventil aufweist, wobei im Inneren des Filtergehäuses mindestens ein Haltemittel zur Vermittlung eines Haltes für den Filterkörper angeordnet ist. Der dort offenbarte Feuchte- und Wärmeaustauscher mit Sprechfunktion ist beispielsweise in ein aus dem Stand der Technik bekanntes Tracheostomapflaster einsetzbar, welches klebend am Hals des Tracheotomierten derart befestigt wird, dass der Feuchte- und Wärmeaustauscher mit Sprechfunktion oberhalb des Tracheostomas angeordnet ist.

Derartige Tracheostomapflaster sind ebenfalls vielfältig aus dem Stand der Technik bekannt. So offenbart DE 603 12 327 T2 ein gattungsgemäßes Tracheostomapflaster, wobei das Pflaster einen Sockel, der an seinen beiden Enden offen ist, um ein Tracheostomaventil oder dergleichen an dem Pflaster anzubringen, einen ringförmigen Flansch, der mit einem proximalen Ende des Sockels an einem Innenumfang des Flansches, der dieses Ende konzentrisch umgibt, durchgehend verbunden ist, wobei der Flansch zur Wand des Sockels hin winklig oder gekrümmt ist, ein ringförmiges Band, das an einer proximalen Seite des Flansches angebracht ist und diese proximale Seite bedeckt, als auch einen Klebstoff an einer proximalen Seite des Bandes umfasst. Dabei ist das ringförmige Band als einfach beschichtetes Klebeband ausgebildet, das durch eine erste ringförmige Verbindungsstelle am Außenumfang des Flansches oder innerhalb von diesem befestigt ist und sich radial über die Kante des Flansches hinaus erstreckt, wobei das ringförmige Band weiterhin durch eine zweite ringförmige Verbindungsstelle, die zwischen dem Außenumfang des Flansches und dem Innenumfang des Flansches von der ersten Verbindungsstelle radial beabstandet angeordnet ist, befestigt ist, wobei eine schützende Decklage den Klebstoff auf dem Band abdeckt.

Nachteilig an dem in der DE 603 12 327 T2 offenbarten Tracheostomapflaster ist die relativ aufwendige Art der Herstellung. Insbesondere muss bei der Herstellung ein aus einem vorteilhafterweise Polyethylenmaterial gebildeter Flansch gezielt an den ersten und zweiten ringförmigen Verbindungsstellen mit einem Klebeband versehen werden, was einen gewissen apparativen Aufwand erfordert. Vorteilhafterweise ist das in der DE 603 12 327 T2 offenbarte Tracheostomapflaster jedoch in der Lage, insbesondere auch bei tief in den Hals des Tracheotomierten eingesogenem Tracheostoma eine hinreichend sichere Abdeckung desselben zur Verfügung zu stellen, so dass beim Einatmen und Ausatmen Luft überwiegend durch das in das Tracheostomapflaster eingesetzte Tracheostomahilfsmittel wie Sprechventil oder Feuchte- und Wärmeaustauscher geführt wird. Nachteilig an dem in der DE 603 12 327 T2 offenbarten Tracheostomapflaster ist, dass der Flansch aus einem zwar relativ weichen Polymer, nämlich Polyethylen, gefertigt ist, dieses jedoch gleichwohl eine gewisse Festigkeit aufweist, die ein Anlegen und Abdichten an die unregelmäßig gestalteten Hautpartien rund um das Tracheostoma des Tracheotomierten herum nur in Teilen zur Verfügung stellen kann.

DE 20 2008 017 105 U1 offenbart eine Vorrichtung zur Stabilisierung eines Tracheostomas zum Befestigen von Pflastern mit Filter, Sprechventilen und Tracheostomiekanülen an Patienten, bei denen z.B. im Zuge einer Laryngektomie eine Tracheostomie durchgeführt wurde.

DE 20 2006 005 101 U1 offenbart eine Anordnung zur Tracheostoma-Stabilisierung in Anwendung mit einem Tracheostoma-Pflaster und einem Stabilisierungsring.

US 2012/0101458 A1 offenbart eine Fistel-Isoliervorrichtung unter Anwendung von vermindertem Druck.

EP 2 497 449 A2 offenbart einen konvex geformten Sperrring zur Verwendung bei Enterostoma-Patienten.

Aufgabe der vorliegenden Erfindung ist es daher, ein Tracheostomapflaster zur Verfügung zu stellen, welches einerseits eine verbesserte Anlage insbesondere bei eingezogenem Tracheostoma zur Verfügung stellt, und zudem vorteilhafterweise einfach herstellbar ist.

Diese Aufgabe wird erfindungsgemäß gelöst durch ein Tracheostomapflaster nach Anspruch 1. Erfindugsgemäß ist das Tracheostomapflaster ein Tracheostomapflaster der eingangs genannten Art mit einem auf der distalen Seite angeordnetem Klebeband, mit einer Aufnahme für ein Tracheostomahilfsmittel, nämlich ein Sprechventil mit einem Feuchte- und Wärmeaustauscher, oder aber einer künstlichen Nase, sowie mit einem ringförmigen Wulst, der auf der proximalen Seite des Pflasters auf einem Teilbereich desselben angeordnet ist, wobei die Aufnahme von dem ringförmigen Wulst umgeben ist. Optional weist eine proximale Oberfläche des Wulstes, gesehen in einem Querschnitt parallel zu einer Mittelachse des Pflasters, im unbenutzten Zustand einen Wendepunkt auf. Der Wulst im Sinne der vorliegenden Erfindung kann dabei auch als Materialanhäufung verstanden werden, die ringförmig um die Aufnahme des Tracheostomapflaster angeordnet ist. Anstatt eines starren Flansches wie in der DE 603 12 327 T2 offenbart, kann durch die Vorsehung eines Wulstes in Form einer Materialanhäufung dessen proximale Oberfläche derart gestaltet werden, dass diese einen engeren Kontakt mit der das Tracheostoma umgebenden Hautpartie des Tracheotomierten zur Verfügung stellt. Da der ringförmige Wulst zudem auf der proximalen Seite des Pflasters auf einem Teilbereich desselben angeordnet ist, kann das erfindungsgemäße Tracheostomapflaster relativ einfach hergestellt werden durch Verbindung eines ringförmigen Wulstes mit einem Klebeband, dessen proximale Seite zumindest teilflächig mit einem Klebemittel versehen ist, insbesondere im nicht vom ringförmigen Wulst abgedeckten Bereich, weiter bevorzugt vollflächig auf dem nicht vom ringförmigen Wulst abgedeckten Bereich der proximalen Oberfläche des Klebebandes. Optional ist eine proximale Oberfläche des Pflasters, die nicht mit dem Wulst verbunden ist, zumindest in Teilbereichen klebefähig ausgebildet. Üblicherweise sind die klebenden Flächen, sei es die proximale Oberfläche des Pflasters, sei es die des Wulstes, mit einem Abdeckband geschützt. Im Sinne der vorliegenden Erfindung ist ein solches mit einem Abdeckband versehenes Tracheostomaplaster als klebefähig anzusprechen. Der ringförmige Wulst ist dabei vorteilhafterweise als Vollmaterial ausgebildet. Vorteilhafterweise ist dabei die distale Seite des Wulstes im Wesentlichen eben ausgebildet. Unter einer ebenen Ausbildung im Sinne der vorliegenden Erfindung ist dabei zu verstehen, dass die distale Seite des Wulstes im Wesentlichen in etwa parallel zu einer glatten Fläche, zur Verfügung gestellt durch die proximale Oberfläche des Klebebandes, ausgebildet ist. Der Wulst ist damit einerseits durch eine ebene Fläche auf der distalen Seite vom Klebeband und dessen proximaler Oberfläche begrenzt, und andererseits von der Aufnahme. Die Aufnahme ist vorteilhafterweise radialsymmetrisch ausgebildet und insbesondere als kreisförmige Öffnung im Tracheostomapflaster, weiter bevorzugt in zentraler Position von diesem, ausgebildet. Das Klebeband im Sinne der vorliegenden Erfindung ist insbesondere als umfassend eine Klebeschicht zu verstehen, die zumindest teilweise die proximale Oberfläche des Pflasters bildet. Bevorzugt bildet das Klebeband vollflächig die proximale Oberfläche des Pflasters mit Ausnahme desjenigen Bereichs, der durch den ringförmigen Wulst gebildet ist.

In einer weiter bevorzugten Ausführungsform ist der Wulst des erfindungsgemäßen Tracheostomapflasters aus einem weichen, druckempfindlichen Material gebildet. Druckempfindlich im Sinne der vorliegenden Erfindung heißt dabei, dass bei Eindrücken des Wulstes das Material bei nachlassendem Druck elastisch oder duroplastisch in die Ausgangsstellung zurückkehrt. Zudem weist das Material des Wulstes die Eigenschaft auf, dass bei Ausübung eines dauerhaften Druckes das Material des Wulstes in angrenzende Bereiche verdrückt wird, wodurch die Abdichtungswirkung gesteigert ist. Zudem ist aufgrund der weichen, druckempfindlichen Ausbildung des Wulstes dessen proximale Oberfläche ausgesprochen gut für Tracheotomierte zu tragen, da sich im angrenzenden Hautbereich um das Tracheostoma des Tracheotomierten herum hierdurch ein angenehmes Tragegefühl erzielen lässt.

In einer weiter bevorzugten Ausführungsform umfasst der Wulst einen in etwa parallel zu dem Klebeband ausgebildeten äußeren Randbereich. Dieser geht weiter bevorzugt versprungfrei, d.h. insbesondere bündig, in das Klebeband über. Weiter bevorzugt ist ein der Aufnahme zugewandter innerer Randbereich des Wulstes ebenfalls in etwa parallel zu dem Klebeband ausgebildet. Dabei bedeutet parallele Ausbildung im Sinne der vorliegenden Erfindung, dass der äußere und innere Randbereich des Wulstes bezogen sind auf ein plan vorliegendes Klebeband. Durch den in etwa parallel zu dem Klebeband ausgebildeten äußeren Randbereich des Wulstes ist einerseits eine gute Verbindung desselben mit dem Klebeband zur Verfügung gestellt, andererseits wird eine verbesserte Dichtwirkung erzielt, da auch angrenzend um das Tracheostoma herum in den dortigen Hautbereichen eine Abdichtungswirkung erzielbar ist. Durch den in etwa parallel zu dem Klebeband ausgebildeten inneren Randbereich des Wulstes hingegen wird eine sichere Aufnahme des Tracheostomahilfsmittel in der Aufnahme zur Verfügung gestellt, insbesondere bei Vorsehung eines in der Aufnahme angeordneten Aufnahmeteils für selbiges. Erfindungsgemäß ist die proximale Oberfläche des Wulstes zumindest in Teilbereichen klebend ausgebildet. Besonders bevorzugt ist die proximale Oberfläche vollflächig klebend ausgebildet. Die klebende Eigenschaft kann dabei durch Vorsehung einer Kleberschicht auf der proximalen Oberfläche des Wulstes vorgesehen sein, sie kann jedoch auch, und zwar bevorzugt, durch das Material des Wulstes selbst zur Verfügung gestellt sein. Durch die Vermittlung klebender Eigenschaften durch die proximale Oberfläche des Wulstes wird der Sitz des Tracheostomapflasters am Tracheostoma des Tracheotomierten verbessert, und insbesondere in Verbindung mit einem aus einem weichen, druckempfindlichen Material hergestellten Vollwulst, der in sich klebende Eigenschaften aufweist, wird die Abdichtungswirkung des erfindungsgemäßen Tracheostomapflaster weiter verbessert.

Erfindungsgemäß ist in der Aufnahme ein Aufnahmeteil mit einer Abschrägung in dem der distalen Seite des Pflasters zugewandten Endbereich des Aufnahmeteils für das Tracheostomahilfsmittel angeordnet. Erfindungsgemäß deckt das Aufnahmeteil eine Innenwandung des Wulstes vollständig ab. Bevorzugt ist eine Außenwandung des Aufnahmeteils, die dem Wulst zugewandt ist, vollständig durch den Wulst abgedeckt. Die Verbindung zwischen Wulst und Aufnahmeteil, insbesondere der Außenwandung des Aufnahmeteils, kann auf vielerlei Weise erfolgen. Bevorzugt erfolgt die Verbindung klebend, kann aber auch durch 1- oder 2-Komponenten-Spritzguß erfolgen. Auch kann diese durch Haltemittel wie Dorne oder ähnliches, die an der Außenwandung des Aufnahmeteils und/oder der Innenwandung des Wulstes angeordnet sind, allein oder zusätzlich in den genannten Verbindungsarten erfolgen. Das Aufnahmeteil kann lediglich als Zylinderabschnitt angeordnet innerhalb der Aufnahme ausgebildet sein, so dass dieses vorteilhafterweise nicht mit dem Tracheostoma oder aber den unmittelbar angrenzenden Hautteilen des Tracheotomierten in Kontakt kommt. Vorteilhafterweise ist das Aufnahmeteil aus einem elastischen Material hergestellt, bevorzugt einem weichen polymeren Material, vorteilhafterweise aus einem Polymermaterial hergestellt aus Polymeren ausgewählt aus einer Gruppe umfassend Polyethylen und/oder Polypropylen. Aber auch andere Polymere sind möglich.

In einer weiter bevorzugten Ausführungsform weist das Aufnahmeteil einen proximalen, insbesondere einen den Außenumfang des Aufnahmeteils überstehenden Rand auf, der mit dem Wulst verbunden ist. Die Verbindung erfolgt dabei vorteilhafterweise mit der proximalen Oberfläche des Wulstes. Weiter vorteilhafterweise erfolgt die Verbindung in einem ringförmigen Bereich unmittelbar angrenzend an die Innenwandung des Wulstes auf dessen proximaler Oberfläche. Durch den proximalen Rand wird die Kontaktfläche des Aufnahmeteiles mit dem Wulst vergrößert, so dass das Aufnahmeteil sicher am Wulst gehalten ist. In einer weiter bevorzugten Ausführungsform geht der proximale Rand des Aufnahmeteiles versprungfrei in den Wulst beziehungsweise dessen proximale Oberflä che über. Bevorzugt schließt ein proximales Ende des Aufnahmeteils bündig mit der proximalen Oberfläche des Wulstes ab. Durch jede der vorstehenden Maßnahmen wird eine glatte proximale Oberfläche, die den in das Tracheostoma umgebenden Hautpartien des Tracheotomierten zugewandt ist, zur Verfügung gestellt, wodurch einerseits eine gute abdichtende Wirkung des erfindungsgemäßen Tracheostomapflasters zur Verfügung gestellt ist, andererseits auch Reizungen in den Hautpartien unmittelbar angrenzend des Tracheostomas der Tracheotomierten vermieden sind. Der Begriff "versprungfrei" ist im Sinne der Erfindung dahingehend zu verstehen, dass ein glatter, bündiger Übergang zwischen den in Rede stehenden Mitteln ohne Ausbildung von Kanten, Rinnen oder ähnlichem erfolgt.

In einer weiter bevorzugten Ausführungsform schließt ein distales Ende des Aufnahmeteiles bündig mit einer distalen Oberfläche des Wulstes ab. Hierdurch weist das erfindungsgemäße Tracheostomapflaster auf der distalen Seite desselben keinerlei Erhöhung, sondern stattdessen ein gleichmäßiges Erscheinungsbild auf, was von Tracheotomierten bevorzugt ist.

Diese und weitere Vorteile werden anhand der folgenden Beispiele näher erläutert. Es zeigen
- Fig. 1:: eine perspektivische Oberansicht eines erfindungsgemäßen Tracheostomapflasters;
- Fig. 2:: eine perspektivische Unteransicht des Tracheostomapflasters gemäß Fig. 1;
- Fig. 3:: eine Seitenansicht des Tracheostomapflasters gemäß Fig. 1; und
- Fig. 4:: eine Unteransicht des Tracheostomapflasters gemäß Fig. 1.

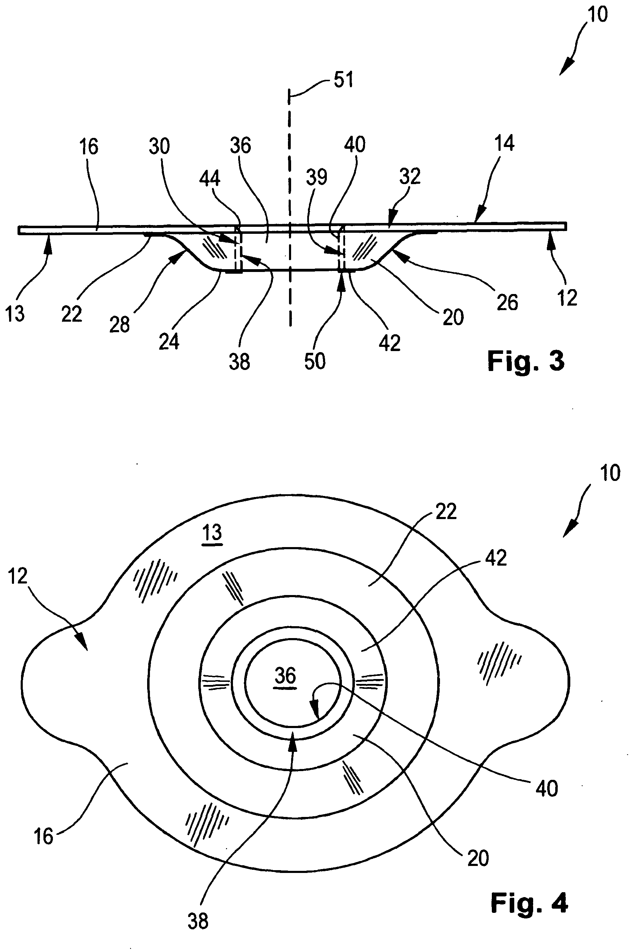

Zunächst sei vorausgeschickt, dass die in den Figuren dargestellte Ausgestaltung des erfindungsgemäßen Tracheostomapflasters nicht einschränkend auszulegen ist. Vielmehr können die dort beschriebenen Merkmale untereinander mit den in der Beschreibung oben beschriebenen Merkmalen zur weiteren Ausgestaltung kombiniert werden. Des Weiteren sei darauf hingewiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen sollen. Gleiche Teile oder Teile mit gleicher Funktion weisen im Folgenden die gleichen Bezugszeichen auf. Es sei insbesondere darauf hingewiesen, dass die in den Figuren gezeigte beispielhafte Ausgestaltung mit einer im Wesentlichen kreisförmigen Grundform mit zwei seitlichen Laschen nur eine der möglichen Ausgestaltungen des erfindungsgemäßen Tracheostomapflasters zeigt. Dieses kann beispielsweise auch eine ovale Grundform, eine rechteckige Grundform oder eine vier-, fünf-, sechs- oder sonstwie mehreckige Grundform aufweisen. Dabei können Laschen vorgesehen sein oder nicht, ebenso wie in den Ausführungsbeispielen nicht gezeigte Hilfslaschen, welche eine Ablösung des Pflasters von einem in den Figuren nicht gezeigten Deckblatt, welches das Klebeband schützt, erleichtern soll. Auch sel darauf hingewiesen, dass die Aufnahme 36 nicht, wie in den Ausführungsbeispielen der vorliegenden Erfindung gezeigt, stets mittig und zentral angeordnet sein muss. Die Aufnahme kann vielmehr insbesondere auf den Kopf des Tracheotomierten zu, aber auch in umgekehrter Richtung verschoben sein. Auch kann die proximale Oberfläche 26 des Wulstes in jeder anderen Form, im Querschnitt gesehen, wie in Fig. 3 gezeigt, ausgestaltet sein, und muss nicht insbesondere einen Wendepunkt aufweisen. Ebenso muss kein proximaler Rand 42 eines Aufnahmeteils 38 vorhanden sein. Dann kann ein proximales Ende 50 des Aufnahmeteils bevorzugt bündig mit einer proximalen Oberfläche des Wulstes 20 abschließen.

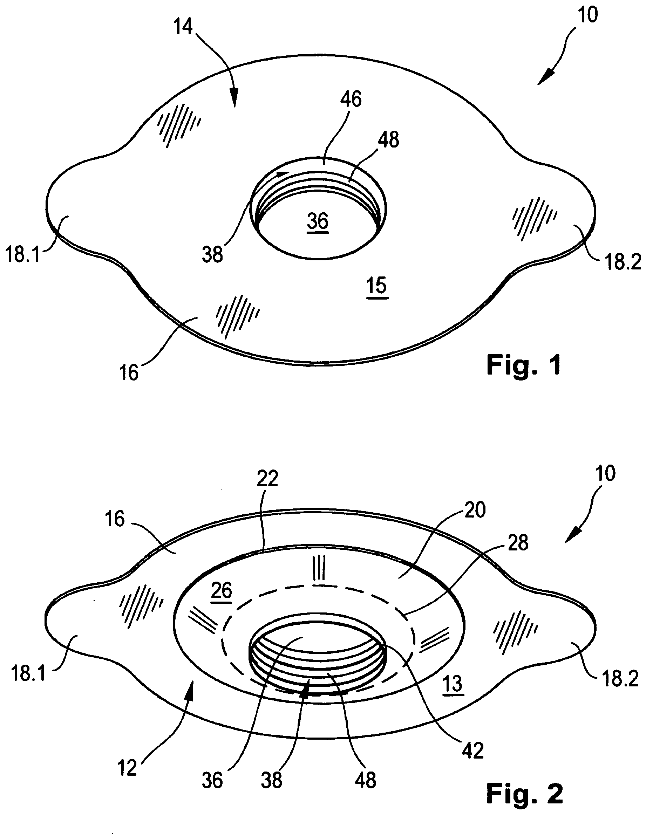

Fig. 1 zeigt nun ein erfindungsgemäßes Tracheostomapflaster 10 mit einer distalen, das heißt vom Körper weggewandten Seite 14 und einer proximalen, das heißt dem Körper zugewandten Seite 12 (Fig. 2). Das Pflaster 10 umfasst ein Klebeband 16. Das Klebeband 16 bildet eine vollflächig klebende proximale Oberfläche 13 des Pflasters 10 in dem Bereich der proximalen Seite 12 desselben, die nicht mit dem Wulst 20 versehen ist. Das Pflaster ist mit einer distalen Oberfläche 15 versehen, die nicht klebend ausgebildet ist. Das Pflaster 10 weist eine im Wesentlichen kreisrunde Grundform auf, wobei an zwei Seiten gegenüberliegend Laschen 18.1 und 18.2 angeordnet sind, welche insbesondere eine Ablösung des Pflasters 10 von der Haut des Tracheotomierten erleichtern sollen.

Das Pflaster 10 weist eine zentral und mittig in diesem angeordnete Aufnahme 36 auf, in welcher ein Aufnahmeteil 38 mit einer Innenwandung 40 angeordnet ist. Das Aufnahmeteil 38 weist etwa auf halber Höhe des aus Fig. 1 nicht ersichtlichen Wulstes 20 eine Verdickung 48 an zumindest der Innenwandung 40 auf, welche einen sicheren Sitz eines in die Aufnahme 36 einzusetzenden Tracheostomahilfsmittels wie einer künstlichen Nase oder einem Sprechventil erleichtert. Das Einsetzen eines solchen Tracheostomahilfsmittels ist weiterhin erleichtert durch die Vorsehung einer Abschrägung 46 in dem der distalen Seite 14 des Pflasters 10 zugewandten Endbereich des Aufnahmeteils 38.

Fig. 2 zeigt das Pflaster 10 gemäß Fig. 1 in einer perspektivischen Unteransicht. Auf der proximalen Seite 12 des Pflasters 10 angeordnet ist ein ringförmiger Wulst 20, welcher umlaufend um die Aufnahme 36 ausgebildet ist. Der Wulst 20 weist dabei einen äußeren Randbereich 22 auf, welcher im Wesentlichen parallel zu dem Klebeband 16 ausgebildet ist (Fig. 3). Weiterhin ist Fig. 2 ein Wendepunkt 28, dort mit einer gestrichelten Linie angedeutet, des Wulstes 20 zu entnehmen.

Darüber hinaus zeigt Fig. 2 eine Ausgestaltung des Aufnahmeteils 38 dahingehend, dass dieses einen proximalen Rand 42 aufweist, welcher mit einer proximalen Oberfläche 26 des Wulstes 20 verbunden ist in einem in etwa ringförmig ausgestalteten Bereich um die Aufnahme 36 herum. Dieser Rand 42 kann in einer alternativen Ausführungsform auch weggelassen werden. Ein proximales Ende 50 des Aufnahmeteils 38 (s. Fig. 3) geht dann bevorzugt versprungfrei bzw. bündig in den Wulst 20 über.

Fig. 3 lässt sich besonders gut die Ausbildung des Tracheostomapflasters 10 gemäß den Fig. 1 und Fig. 2 entnehmen, insbesondere in Hinblick auf die Ausgestaltung des Wulstes 20. Die proximale Oberfläche 26 des Wulstes 20 weist den Wendepunkt 28 auf. Der Begriff Wendepunkt ist dabei im Sinne der vorliegenden Erfindung im mathematischen Sinne zu verstehen. Der Wendepunkt 28 befindet sich mittig zwischen dem äußeren Randbereich 22 und einem inneren Randbereich 24 des Wulstes 20, welcher jeweils in etwa parallel zu dem Klebeband 16 ausgebildet sind. Der Wulst 20 weist eine Innenwandung 30 auf, an welcher unmittelbar angeordnet das Aufnahmeteil 38 ist. Das Aufnahmeteil 38 weist eine Außenwandung 39 auf, die vollflächig durch den Wulst 20 abgedeckt ist. Das Aufnahmeteil 38 weist den proximalen Rand 42 auf, mit welchem der Wulst 20 umgriffen wird, und der unmittelbar am Wulst 20 anliegt und mit diesem verbunden ist. Dabei geht der proximale Rand 42 versprungfrei in das Material des Wulstes 20 beziehungsweise dessen Oberfläche 26 über.

Der Wendepunkt 28 ist definiert durch einen Querschnitt durch das Pflaster 10, nämlich einen Querschnitt durch dieses parallel zu einer Mittelachse 51 des Pflasters.

Das Aufnahmeteil 38 weist eine innere, zylinderabschnittsförmige Wandung 40 auf mit einem distalen Ende 44 und dem proximalen Ende 50. Das distale Ende 44 des Aufnahmeteiles 38 schließt dabei bündig mit einer distalen Oberfläche 32 des Wulstes 20 ab. Das distale Ende 44 kann dabei vom Klebeband 16 überdeckt sein.

Fig. 4 zeigt in einer Unteransicht das Tracheostomapflaster 10 gemäß den Fig. 1 bis Fig. 3, wobei die Ausgestaltung des Wulstes 20 mit dem äußeren Rand 22 gut verdeutlicht ist, ebenso wie die Anordnung des Aufnahmeteiles 38 in der Aufnahme 36 mit dem proximalen Rand 42.

In Fig. 1 bis Fig. 4 ist dabei ein Abdeckband, welches auf der proximalen Seite 12 des Pflasters 10 anordbar ist, zumindest im Bereich der freiliegenden Unterseite des Klebebandes 16, welches bevorzugt vollflächig klebend ausgebildet ist und die proximale Oberfläche 13 bildet, nicht gezeigt. Das Abdeckband kann insbesondere auch bei der erfindungsgemäßen zumindest teilweise klebenden Ausgestaltung der proximalen Oberfläche 26 des Wulstes 20 selbigen abdecken. Eine Abdeckung der proximalen Oberfläche 26 des Wulstes 20 kann jedoch auch allein zum Schutz derselben erfolgen.

Durch die vorliegende Erfindung wird ein Tracheostomapflaster zur Verfügung gestellt, welches vorteilhafterweise eine gute Anlage und Abdichtwirkung in dem unmittelbar dem Tracheostoma angrenzenden Hautbereich eines Tracheotomierten aufweist, und zudem einfach herstellbar ist.

## Patentansprüche

1. Tracheostomapflaster (10) mit einer proximalen, dem Körper zugewandten, und einer distalen, dem Körper abgewandten Seite (12, 14), einem auf der distalen Seite (14) angeordneten Klebeband (16), mit einer Aufnahme (36) für ein Tracheostomahilfsmittel, nämlich ein Sprechventil oder eine künstliche Nase, sowie mit einem ringförmigen Wulst (20), der auf der proximalen Seite (12) auf einem Teilbereich derselben angeordnet ist, wobei die Aufnahme (36) von dem ringförmigen Wulst (20) umgeben ist und eine proximale Oberfläche (26) des Wulstes (20) zumindest in Teilbereichen klebend ausgebildet ist und wobei in der Aufnahme (36) ein Aufnahmeteil (38) mit einer Abschrägung (46) in dem der distalen Seite (14) des Pflasters (10) zugewandten Endbereich des Aufnahmeteils (38) für das Tracheostomahilfsmittel angeordnet ist, wobei das Aufnahmeteil (38) eine Innenwandung (30) des Wulstes (20) vollständig abdeckt.

2. Tracheostomapflaster gemäß Anspruch 1, **dadurch gekennzeichnet, dass** der Wulst (20) einen in etwa parallel zu dem Klebeband (16) ausgebildeten äußeren Randbereich (22) umfasst.

3. Tracheostomapflaster gemäß einem der vorhergehenden Ansprüche, **dadurch gekennzeichnet, dass** die proximale Oberfläche (26) des Wulstes (20), gesehen in einem Querschnitt parallel zu einer Mittelachse (51) des Pflasters (10), im unbenutzten Zustand einen Wendepunkt (28) aufweist.

4. Tracheostomapflaster gemäß einem der vorhergehenden Ansprüche, **dadurch gekennzeichnet, dass** der Wulst (20) aus einem weichen, druckempfindlichen Material gebildet ist.

5. Tracheostomapflaster gemäß einem der vorhergehenden Ansprüche, **dadurch gekennzeichnet, dass** der Wulst (20) auf der distalen Seite (14) im Wesentlichen eben ausgebildet ist.

6. Tracheostomapflaster gemäß einem der vorhergehenden Ansprüche, **dadurch gekennzeichnet, dass** ein der Aufnahme (36) zugeordneter innerer Randbereich (24) des Wulstes (20) in etwa parallel zu dem Klebeband (16) ausgebildet ist.

7. Tracheostomapflaster gemäß einem der vorhergehenden Ansprüche, **dadurch gekennzeichnet, dass** das Aufnahmeteil (38) einen proximalen Rand (42) aufweist, der mit dem Wulst (20) verbunden ist.

8. Tracheostomapflaster gemäß Anspruch 7, **dadurch gekennzeichnet, dass** der proximale Rand (42) versprungfrei in den Wulst (20) übergeht.

9. Tracheostomapflaster gemäß einem der vorhergehenden Ansprüche, **dadurch gekennzeichnet, dass** das Aufnahmeteil (38) aus einem elastischen Material herstellt ist.

10. Tracheostomapflaster gemäß einem der vorhergehenden Ansprüche, **dadurch gekennzeichnet, dass** ein distales Ende (44) des Aufnahmeteiles (38) bündig mit einer distalen Oberfläche (32) des Wulstes (20) abschließt.

11. Tracheostomapflaster gemäß einem der vorhergehenden Ansprüche, **dadurch gekennzeichnet, dass** ein proximales Ende (50) des Aufnahmeteils (38) bündig mit der proximalen Oberfläche (26) des Wulstes (20) abschließt.

12. Tracheostomapflaster gemäß einem der vorhergehenden Ansprüche, **dadurch gekennzeichnet, dass** eine Außenwandung (39) des Aufnahmeteils (38) vollständig durch den Wulst (20) abgedeckt ist.

13. Tracheostomapflaster gemäß einem der vorhergehenden Ansprüche, **dadurch gekennzeichnet, dass** eine proximale Oberfläche (13) des Pflasters (10), die nicht mit dem Wulst (20) verbunden ist, zumindest in Teilbereichen klebefähig abgebildet ist.

## Claims

1. Tracheostoma plaster (10) having a proximal side facing the body, and a distal side (12, 14) facing away from the body, an adhesive strip (16) arranged on the distal side (14), having a receptacle (36) for a tracheostoma aid, namely a speech valve or an artificial nose, as well as an annular bead (20) arranged on a portion of the proximal side (12), wherein the receptacle (36) is surrounded by the annular bead (20) and a proximal surface (26) of the bead (20) is rendered adhesive at least in portions, and wherein a receiving part (38) is arranged in the receptacle (36), which has a chamfering (46) in the end portion of the receptacle (36) for the tracheostoma aid, facing the distal side (14) of the plaster (10), wherein the receiving part (38) completely covers an inner wall (30) of the bead (20).

2. Tracheostoma plaster according to claim 1, **characterized in that** the bead (20) comprises an outer marginal area (22) running approximately parallel to the adhesive strip (16).

3. Tracheostoma plaster according to one of the preceding claims, **characterized in that** in an unused state the proximal surface (26) of the bead (20), viewed in cross-section parallel to a central axis (51) of the plaster (10), has a turning point (28).

4. Tracheostoma plaster according to one of the preceding claims, **characterized in that** the bead (20) consists of a soft, pressure-sensitive material.

5. Tracheostoma plaster according to one of the preceding claims, **characterized in that** the bead (20) has a basically planar design on the distal side (14).

6. Tracheostoma plaster according to one of the preceding claims, **characterized in that** an inner marginal area (24) of the bead (20) attached to the receptacle (36), is configured approximately parallel to the adhesive strip (16).

7. Tracheostoma plaster according one of the preceding claims, **characterized in that** the receiving part (38) has a proximal edge (42), which is connected with the bead (20).

8. Tracheostoma plaster according to claim 7, **characterized in that** the proximal edge (42) merges without any projection into the bead (20).

9. Tracheostoma plaster according to one of the preceding claims, **characterized in that** the receiving part (38) is made of a flexible material.

10. Tracheostoma plaster according to one of the preceding claims, **characterized in that** a distal end (44) of the receiving part (38) forms a flush seal with a distal surface (32) of the bead (20).

11. Tracheostoma plaster according to one of the preceding claims, **characterized in that** a proximal end (50) of the receiving part (38) forms a flush seal with the proximal surface (26) of the bead (20).

12. Tracheostoma plaster according to one of the preceding claims, **characterized in that** an outer wall (39) of the receiving part (38) is completely covered by the bead (20).

13. Tracheostoma plaster according to one of the preceding claims, **characterized in that** a proximal surface (13) of the plaster (10), which is not connected with the bead (20), is rendered adhesive at least in portions.

## Revendications

1. Pansement pour trachéotomie (10) avec un côté proximal (12) tourné vers le corps et un côté distal (14) détourné du corps, avec une bande adhésive (16) disposée sur le côté distal (14), avec un logement (36) pour un accessoire de trachéotomie, à savoir une valve phonique ou un nez artificiel, ainsi qu'avec un bourrelet annulaire (20), qui est disposé sur le côté proximal (12) sur une zone partielle de celui-ci, dans lequel le logement (36) est entouré par le bourrelet annulaire (20) et une surface proximale (26) du bourrelet (20) est rendue adhésive au moins dans des zones partielles et dans lequel une pièce de réception (38) pour l'accessoire de trachéotomie, présentant un biseau (46) dans la région d'extrémité de la pièce de réception (38) tournée vers le côté distal (14) du pansement (10), est disposée dans le logement (36), dans lequel la pièce de réception (38) recouvre entièrement une paroi intérieure (30) du bourrelet (20).

2. Pansement pour trachéotomie selon la revendication 1, **caractérisé en ce que** le bourrelet (20) comprend une région de bord extérieure (22) sensiblement parallèle à la bande adhésive (16).

3. Pansement pour trachéotomie selon l'une quelconque des revendications précédentes, **caractérisé en ce que** la surface proximale (26) du bourrelet (20), vue en coupe transversale parallèle à un axe central (51) du pansement (10), présente à l'état non utilisé un point d'inflexion (28).

4. Pansement pour trachéotomie selon l'une quelconque des revendications précédentes, **caractérisé en ce que** le bourrelet (20) est formé en un matériau souple, sensible à la pression.

5. Pansement pour trachéotomie selon l'une quelconque des revendications précédentes, **caractérisé en ce que** le bourrelet (20) est de forme essentiellement plane sur le côté distal (14).

6. Pansement pour trachéotomie selon l'une quelconque des revendications précédentes, **caractérisé en ce qu'**une région de bord intérieure (24) du bourrelet (20) associée au logement (36) est sensiblement parallèle à la bande adhésive (16).

7. Pansement pour trachéotomie selon l'une quelconque des revendications précédentes, **caractérisé en ce que** la pièce de réception (38) présente un bord proximal (42), qui est relié au bourrelet (20).

8. Pansement pour trachéotomie selon la revendication 7, **caractérisé en ce que** le bord proximal (42) se prolonge sans retrait dans le bourrelet (20).

9. Pansement pour trachéotomie selon l'une quelconque des revendications précédentes, **caractérisé en ce que** la pièce de réception (38) est fabriquée en un matériau élastique.

10. Pansement pour trachéotomie selon l'une quelconque des revendications précédentes, **caractérisé en ce qu'**une extrémité distale (44) de la pièce de réception (38) se termine à fleur d'une surface distale (32) du bourrelet (20).

11. Pansement pour trachéotomie selon l'une quelconque des revendications précédentes, **caractérisé en ce qu'**une extrémité proximale (50) de la pièce de réception (38) se termine à fleur de la surface proximale (26) du bourrelet (20).

12. Pansement pour trachéotomie selon l'une quelconque des revendications précédentes, **caractérisé en ce qu'**une paroi extérieure (39) de la pièce de réception (38) est recouverte entièrement par le bourrelet (20).

13. Pansement pour trachéotomie selon l'une quelconque des revendications précédentes, **caractérisé en ce qu'**une surface proximale (13) du pansement (10), qui n'est pas reliée du bourrelet (20), est rendue adhésive au moins dans des zones partielles.
